# FASCICULE DE BREVET EUROPEEN

(11) **EP 2 024 312 B1**
(45) Date de publication et mention de la délivrance du brevet: **31.12.2014**
(21) Numéro de dépôt: 07766012.4
(22) Date de dépôt: 09.05.2007
(51) Int. Cl.: C07C 17/21, C07C 19/08

(54) **PROCEDE DE FABRICATION DU PENTAFLUOROETHANE**
VERFAHREN ZUR HERSTELLUNG VON PENTAFLUORETHAN
METHOD FOR PRODUCING PENTAFLUOROETHANE

(30) Priorité: 30.05.2006 FR 0604783
(43) Date de publication de la demande: 18.02.2009
(73) Titulaire: ARKEMA FRANCE, 92700 Colombes (FR)
(72) Inventeur: COLLIER, Bertrand, 69230 Saint-genis-laval (FR); CAVALLINI, Géraldine, 69360 Saint-symphorien D'ozon (FR); BOUSSAND, Béatrice, 69110 Sainte Foy Les Lyon (FR)
(74) Mandataire: Dang, Doris
(86) Numéro de dépôt international: PCT/FR2007/051232
(87) Numéro de publication internationale: WO 2007/138209

(56) Documents cités:
- EP-B1- 0 754 170
- US-A- 4 123 448
- US-A- 5 395 996

## Description

La présente invention concerne un procédé de fabrication du pentafluoroéthane et a plus particulièrement pour objet un procédé de fabrication du pentafluoroéthane en faisant réagir du perchloroéthylène (PER) avec de l'acide fluorhydrique (HF) en phase gazeuse en présence d'un catalyseur.

La réaction de fluoration du perchloroéthylène avec de l'HF en phase gazeuse en présence d'un catalyseur est connue. Elle conduit en général à la formation du 2,2-dichloro-1,1,1-trifluoroéthane (123), 2-chloro-1,1,1,2-tetrafluoroéthane (124) et pentafluoroéthane (125) avec le 123 comme produit majoritaire.

Ces composés (globalement désignés ci-après par l'expression «série 120 ») peuvent être utilisés soit comme substituts des chlorofluorocarbures (CFC) dans les domaines des mousses (agents gonflants et isolants), des aérosols (agents propulseurs) ou dans la réfrigération, soit comme intermédiaires de synthèse de ces substituts. On recherche actuellement des procédés performants pour la production industrielle du pentafluoroéthane.

La réaction de fluoration du perchloroéthylène en pentafluoroéthane étant une réaction fortement exothermique (25 - 30 Kcal/mol), sa mise en oeuvre à l'échelle industrielle pose de nombreux problèmes : la réaction est difficile à contrôler, le catalyseur se dégrade et des sous-produits sont formés en quantité importante.

Le document EP 687660 divulgue un procédé de fabrication du pentafluoroéthane dans lequel des réactions sont montées dans deux zones réactionnelles comprenant une première zone réactionnelle dans laquelle on fait réagir du perchloroéthylène avec du fluorure d'hydrogène en phase gazeuse en présence d'un catalyseur sous une pression comprise entre 3 bar absolu et 30 bar absolu et à une température comprise entre 200°C et 450°C, et une seconde zone réactionnelle dans laquelle on fait réagir le 2,2-dichloro-1,1,1-trifluoroéthane et/ou 2-chloro-1,1,1,2-tetrafluoroéthane contenu dans les gaz produits dans la première zone réactionnelle avec du fluorure d'hydrogène en phase vapeur en présence d'un catalyseur sous une pression qui n'excède pas 5 bar absolu et à une température comprise entre 250°C et 500°C, ladite première zone réactionnelle étant maintenue à une pression supérieure à celle de la seconde zone réactionnelle.

Le document EP 754170 décrit un procédé de production de pentafluoroéthane, qui comprend (i) la mise en contact de perchloroéthylène avec du fluorure d'hydrogène en phase gazeuse en présence d'un premier catalyseur de fluoration comprenant de l'oxyde chromique de façon à former un courant de produit comprenant un hydrochlorofluoroéthane de formule C₂H₁CI₁₊ₓF_{1+y} où x et y sont chacun indépendamment 0, 1, 2 ou 3, étant entendu que x + y est 3, et (ii) la mise en contact du courant de produit de l'étape (i) avec du fluorure d'hydrogène en phase gazeuse et en présence d'un second catalyseur de fluoration comprenant du zinc et/ou du nickel ou un composé de zinc et/ou nickel déposé sur de l'oxyde chromique, du fluorure de chrome ou de l'oxyfluorure de chrome de façon à produire du pentafluoroéthane.

La présente invention fournit un procédé de fabrication du pentafluoroéthane à partir du perchloroéthylène qui permet de résoudre en partie ou en totalité les inconvénients précités.

Le procédé selon la présente invention est caractérisé en ce qu'il comprend une étape au cours de laquelle du perchloroéthylène réagit avec de l'acide fluorhydrique en phase gazeuse en présence d'un catalyseur dans un réacteur multiétagé adiabatique.

La température à l'entrée du premier étage du réacteur adiabatique est comprise entre 280 et 350°C, avantageusement comprise entre 320 et 340°C. La température à l'entrée des étages suivants du réacteur adiabatique peut également varier entre 280 et 350°C, de préférence entre 320 et 340 °C.

Au sein d'un même étage, la température à l'entrée est en général inférieure à celle en sortie. D'autre part, la température à l'entrée de l'étage précédent est de préférence inférieure à celle à l'entrée de l'étage suivant.

Le procédé, selon la présente invention, est avantageusement mis en oeuvre de manière à ce que la température dans le réacteur multiétagé adiabatique n'excède pas 410°C, de préférence n'excède pas 380 °C.

Le réacteur adiabatique peut comprendre entre 2 et 6 étages. Un nombre d'étages compris entre 2 et 4 est toutefois préféré.

Bien que l'étape de fluoration peut être mise en oeuvre dans une large gamme de pression, on préfère opérer à une pression absolue comprise entre 1 et 10 bar et avantageusement comprise entre 1 et 4 bar.

Les réactifs sont préalablement vaporisés avant l'étape de fluoration et de préférence, vaporisés et préchauffés à une température inférieure à celle du réacteur adiabatique et avantageusement à une température comprise entre 320 et 340 °C.

La majeur partie, de préférence plus de 90 % en poids, des réactifs vaporisés et préchauffés sont introduits à l'entrée du premier étage du réacteur. Une faible partie, de préférence moins de 10 % en poids, des réactifs vaporisés et préchauffés, de préférence préchauffés à une température comprise entre 150 et 200°C, sont introduits au niveau inter-étage pour mieux contrôler la température du réacteur adiabatique De préférence, les réactifs préchauffés introduits à l'entrée du premier étage du réacteur sont à une température supérieure à celle des réactifs introduits au niveau inter-étage.

Selon un mode de réalisation de l'invention, le flux sortant du réacteur multiétagé adiabatique est soumis à une étape de séparation pour obtenir une fraction de produits légers, comme par exemple le pentafluoroéthane et le chlorure d'hydrogène et une fraction de produits lourds, comme par exemple le perchloroéthylène non réagi, l'acide fluorhydrique non réagi, les composés intermédiaires, notamment le 2,2-dichloro-1,1,1-trifluoroéthane (123), et le 2-chloro-1,1,1,2-tetrafluoroéthane (124). La fraction de produits lourds, après vaporisation et préchauffage, est ensuite recyclée au réacteur.

Lorsque la fraction de produits lourds est recyclée au réacteur, outre le perchloroéthylène, les composés intermédiaires tels que le 2,2-dichloro-1,1,1-trifluoroéthane et le 2-chloro-1,1,1,2-tetrafluoroéthane réagissent également avec de l'acide fluorhydrique.

Le procédé peut être mis en oeuvre en continu ou discontinu, mais on préfère opérer en continu.

Le ratio molaire HF/ réactifs organiques est en général compris entre 5 et 50, de préférence compris entre 10 et 30 et avantageusement compris entre 10 et 20.

Le temps de contact, calculé comme étant le temps de passage des gaz (dans les conditions réactionnelles) à travers le volume de catalyseur est de préférence compris entre 5 et 40 s, avantageusement compris entre 10 et 20 s.

Lorsque la pression de l'étape de fluoration est inférieure à 4 bar absolu, il est souvent avantageux de comprimer les produits de la réaction avant de les soumettre à l'étape de séparation. Cette compression peut être effectuée à l'aide d'un compresseur et permet d'effectuer la séparation, lorsqu'il s'agit par exemple d'une distillation, dans des conditions énergétiques favorables. En outre, elle permet de récupérer plus de 99 % de l'acide fluorhydrique non réagi.

Tout catalyseur de fluoration peut convenir au procédé de la présente invention. Le catalyseur utilisé comprend de préférence les oxydes, halogénures, oxyhalogénures ou sels minéraux du chrome, d'aluminium, de cobalt, de manganèse, de nickel, de fer ou de zinc, et pouvant être supporté. A titre d'exemple, on peut citer l'alumine, le fluorure d'aluminium, ou l'oxyfluorure d'aluminium comme support.

On utilise de préférence un catalyseur à base d'oxyde de chrome (Cr₂O₃) incluant éventuellement un autre métal de degré d'oxydation supérieur à zéro et sélectionné parmi le Ni, Co, Mn et Zn. Avantageusement ce catalyseur peut être supporté sur de l'alumine, de l'aluminium fluoré ou de l'oxyfluorure d'aluminium.

Un catalyseur mixte composé d'oxydes, d'halogénures et/ou d'oxyhalogénure de nickel et de chrome déposés sur un support constitué de fluorure d'aluminium ou d'un mélange de fluorures d'aluminium et d'alumine convient tout particulièrement au procédé de la présente invention.

Ce catalyseur peut être préparé de façon connue en soi à partir d'une alumine activée. Celle-ci peut dans une première étape être transformée en fluorure d'aluminium ou en mélange de fluorure d'aluminium et d'alumine, par fluoration à l'aide d'acide fluorhydrique en présence éventuellement d'air ou d'un inerte tel que l'azote à une température en général comprise entre 200 et 450°C, de préférence entre 250 et 400°C. Le support est ensuite imprégné à l'aide de solutions aqueuses de sels de chrome et de nickel ou à l'aide de solutions aqueuses d'acide chromique, de sel de nickel et d'un réducteur de chrome comme le méthanol.

Lorsque l'on utilise l'acide chromique (CrO₃) comme précurseur du chrome, on peut réduire ce chrome par tout moyen connu de l'homme de l'art sous réserve que la technique utilisée ne nuise pas aux propriétés du catalyseur et donc à son activité. Le réducteur préféré est le méthanol.

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur mixte utilisé dans le procédé selon l'invention peut aussi être préparé par imprégnation directe de l'alumine par des solutions des composés de chrome et de nickel ci-dessus mentionnées. Dans ce cas, la transformation d'au moins une partie de l'alumine en fluorure d'aluminium s'effectue lors de l'étape d'activation du catalyseur.

Les alumines activées à utiliser pour la préparation du catalyseur mixte sont des produits bien connus, disponibles dans le commerce. Elles sont généralement préparées par calcination d'hydrates d'alumine à une température comprise entre 300 et 800°C. Les alumines activées, utilisables dans le cadre de la présente invention, peuvent contenir des teneurs importantes (jusqu'à 100 ppm) de sodium sans que cela nuise à l'activité catalytique.

Le catalyseur mixte peut contenir en masse de 0,5 à 20 % de chrome et de 0,5 à 20 % de nickel et, de préférence, entre 2 et 10 % de chacun des métaux dans un rapport atomique nickel/chrome compris entre 0,5 et 5, de préférence voisin de 1.

Avant utilisation dans la réaction de fluoration du perchloroéthylène, le solide catalytique est préalablement soumis à une opération d'activation.

Ce traitement réalisé soit « in situ » (dans le réacteur de fluoration) soit dans un appareillage adéquat comprend généralement les étapes suivantes :
- séchage à basse température en présence d'air ou d'azote,
- séchage à haute température (250 à 450°C, de préférence 300 à 350°C) sous azote ou sous air,
- fluoration à basse température (180 à 350°C) au moyen d'un mélange d'acide fluorhydrique et d'azote, en contrôlant la teneur en HF de façon que la température ne dépasse pas 350°C et finition sous courant d'acide fluorhydrique pur ou dilué par de l'azote à une température pouvant aller jusqu'à 450°C.

Bien que cela ne soit pas nécessaire pour la réaction de fluoration, il peut être judicieux d'introduire, avec les réactifs, de l'oxygène à faible teneur. Cette teneur peut varier selon les conditions opératoires entre 0,02 et 1 % molaire par rapport aux réactifs entrant dans le réacteur. L'oxygène pourra être introduit, soit pur soit dilué dans un gaz inerte tel que l'azote, dans le réacteur. L'oxygène pourra également être introduit sous forme d'air. L'introduction d'oxygène, quelle que soit la forme adoptée, pourra se faire de manière continue ou séquentielle.

Le réacteur adiabatique peut être construit à partir des matériaux résistant aux milieux corrosifs comprenant l'acide fluorhydrique, par exemple HASTELLOY et INCONEL.

Le procédé selon la présente invention permet de bien maîtriser l'exothermicité de la réaction et donc d'éviter en partie ou en totalité les inconvénients tels que la désactivation prématurée du catalyseur. En outre, le procédé permet d'obtenir une très forte productivité en pentafluoroéthane. De plus, ce procédé permet de recycler les réactifs non réagis et les composés intermédiaires sans purification préalable.

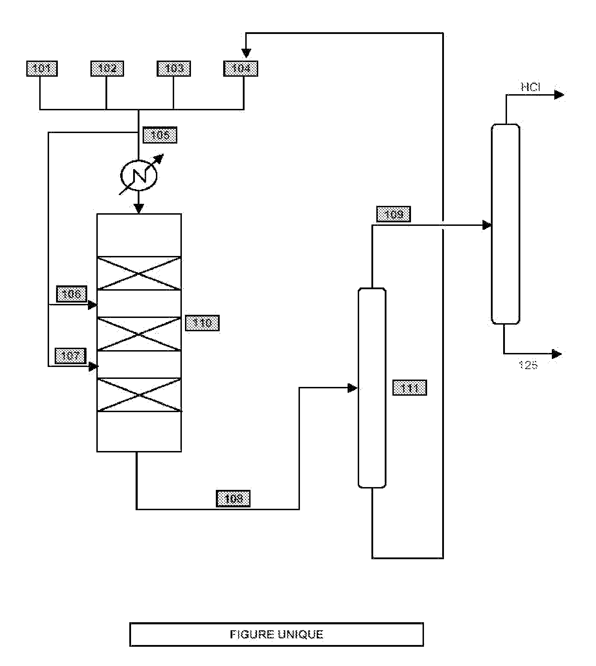

En référence à la figure unique est décrit un mode de réalisation de l'invention. On alimente un réacteur adiabatique (110), constitué de trois étages et contenant un catalyseur à base d'oxyde de chrome, éventuellement supporté, préalablement activé, à l'aide d'un flux gazeux (105) comprenant d'une part du perchoroéthylène (101), de l'acide fluorhydrique (102) et éventuellement de l'air (103), et d'autre part de l'HF, du PER et des composés intermédiaires (majoritairement du 2,2-dichloro-1,1,1-trifluoroéthane et du 2-chloro-1,1,1,2-tetrafluoroéthane) recyclés en provenance du flux (104). Le flux gazeux (105) est préchauffé à 300°C avant l'introduction dans le réacteur et la température à l'entrée du premier étage est maintenue entre 320 et 340° C. Le deuxième étage du réacteur est alimenté par le flux gazeux provenant du premier étage et éventuellement par le flux gazeux (106) comprenant les réactifs préchauffés à 180°C et éventuellement de l'air. La température à l'entrée du deuxième étage est également maintenue entre 320 et 340° C. Le troisième étage du réacteur est alimenté par le flux gazeux issu du deuxième étage et éventuellement par le flux gazeux (107) comprenant les réactifs préchauffés à 180°C et éventuellement de l'air. La température à l'entrée du troisième étage est également maintenue entre 320 et 340°C. Le flux gazeux (108) sortant du réacteur est envoyé à la colonne de distillation (111), pour donner en haut, une fraction de produits légers (109) comprenant notamment du pentafluoroéthane et de l'HCl et en bas, une fraction de produits lourds comprenant de l'HF, du PER et des composés intermédiaires (majoritairement du 2,2-dichloro-1,1,1-trifluoroéthane et du 2-chloro-1,1,1,2-tetrafluoroéthane). La fraction de produits lourds quitte la colonne de distillation par le bas et est ensuite recyclée au réacteur tandis que la fraction de produits légers est soumise à une étape de distillation pour séparer l'HCl du pentafluoroéthane. Le pentafluoroéthane est ensuite purifié.

Pendant toute la durée de la réaction, la température du réacteur multiétagé est maintenue au plus à 380° C et la pression absolue est d'environ 3 bar.

## Revendications

1. Procédé de fabrication du pentafluoroéthane comprenant (i) une étape au cours de laquelle du perchloroéthylène et éventuellement du 2,2-dichloro-1,1,1-trifluoroéthane et/ou du 2-chloro-1,1,1,2-tetrafluoroéthane réagit ou réagissent avec de l'acide fluorhydrique en phase gazeuse en présence d'un catalyseur dans un réacteur multiétagé adiabatique, la température à l'entrée du premier étage du réacteur adiabatique étant comprise entre 280 et 350°C, et éventuellement (ii) une étape de séparation du flux sortant de l'étape (i) pour donner une fraction de produits légers et une fraction de produits lourds.

2. Procédé selon la revendication 1 **caractérisé en ce que** la température à l'entrée du premier étage du réacteur adiabatique est comprise entre 320 et 340°C.

3. Procédé selon l'une quelconque des revendications 1 à 2 **caractérisé en ce que** la température dans le réacteur n'excède pas 410°C, de préférence n'excède pas 380°C.

4. Procédé selon l'une quelconque des revendications 1 à 3 **caractérisé en ce que** la pression absolue de l'étape de fluoration est comprise entre 1 et 10 bar et de préférence comprise entre 1 et 4 bar.

5. Procédé selon l'une quelconque des revendications 1 à 4 **caractérisé en ce que** la fraction de produits lourds est recyclée au réacteur.

6. Procédé selon l'une quelconque des revendications 1 à 5 **caractérisé en ce que** le ratio molaire HF/ réactifs organiques est compris entre 5 et 50, de préférence entre 10 et 30 et avantageusement entre 10 et 20.

7. Procédé selon l'une quelconque des revendications 1 à 6 **caractérisé en ce que** le catalyseur est à base d'oxyde de chrome (Cr₂O₃) incluant éventuellement un autre métal de degré d'oxydation supérieur à zéro et sélectionné parmi le Ni, Co, Mn et Zn.

## Patentansprüche

1. Verfahren zur Herstellung von Pentafluorethan, umfassend (i) einen Schritt, bei dem Perchlorethylen und gegebenenfalls 2,2-Dichlor-1,1,1-trifluorethan und/oder 2-Chlor-1,1,1,2-tetrafluorethan in der Gasphase in Gegenwart eines Katalysators in einem adiabatischen Mehrstufenreaktor mit Fluorwasserstoffsäure reagiert bzw. reagieren, wobei die Temperatur am Eingang der ersten Stufe des adiabatischen Reaktors zwischen 280 und 350°C liegt, und gegebenenfalls (ii) einen Schritt der Trennung des Stroms aus Schritt (i) zum Erhalt einer Fraktion leichter Produkte und einer Fraktion schwerer Produkte.

2. Verfahren nach Anspruch 1, **dadurch gekennzeichnet, dass** die Temperatur am Eingang der ersten Stufe des adiabatischen Reaktors zwischen 320 und 340°C liegt.

3. Verfahren nach einem der Ansprüche 1 bis 2, **dadurch gekennzeichnet, dass** die Temperatur im Reaktor nicht über 410°C und vorzugsweise nicht über 380°C liegt.

4. Verfahren nach einem der Ansprüche 1 bis 3, **dadurch gekennzeichnet, dass** der Absolutdruck des Fluorierungsschritts zwischen 1 und 10 bar und vorzugsweise zwischen 1 und 4 bar liegt.

5. Verfahren nach einem der Ansprüche 1 bis 4, **dadurch gekennzeichnet, dass** die Fraktion schwerer Produkte in den Reaktor zurückgeführt wird.

6. Verfahren nach einem der Ansprüche 1 bis 5, **dadurch gekennzeichnet, dass** das Molverhältnis von HF zu organischen Reaktanten zwischen 5 und 50, vorzugsweise zwischen 10 und 30 und vorteilhafterweise zwischen 10 und 20 liegt.

7. Verfahren nach einem der Ansprüche 1 bis 6, **dadurch gekennzeichnet, dass** der Katalysator auf Chromoxid (Cr₂O₃), gegebenenfalls mit einem anderen Metall, das eine Oxidationsstufe größer null aufweist und aus Ni, Co, Mn und Zn ausgewählt ist, basiert.

## Claims

1. Method for producing pentafluoroethane, comprising (i) a step during which perchloroethylene and, optionally, 2,2-dichloro-1,1,1-trifluoroethane and/or 2-chloro-1,1,1,2-tetrafluoroethane react(s) with hydrofluoric acid, in the gas phase, in the presence of a catalyst in an adiabatic multi-stage reactor, the temperature at the inlet of the first stage of the adiabatic reactor being between 280 and 350°C, and optionally (ii) a step of separating the stream produced in step (i) in order to give a fraction of light products and a fraction of heavy products.

2. Method according to Claim 1, **characterized in that** the temperature at the inlet of the first stage of the adiabatic reactor is between 320 and 340°C.

3. Method according to either one of Claims 1 and 2, **characterized in that** the temperature in the reactor does not exceed 410°C, preferably does not exceed 380°C.

4. Method according to any one of Claims 1 to 3, **characterized in that** the absolute pressure of the fluorination step is between 1 and 10 bar, and preferably between 1 and 4 bar.

5. Method according to any one of Claims 1 to 4, **characterized in that** the fraction of heavy products is recycled to the reactor.

6. Method according to any one of Claims 1 to 5, **characterized in that** the HF/organic reactants molar ratio is between 5 and 50, preferably between 10 and 30, and advantageously between 10 and 20.

7. Method according to any one of Claims 1 to 6, **characterized in that** the catalyst is a chromium oxide (Cr₂O₃)-based catalyst optionally including another metal in an oxidation state above zero and selected from Ni, Co, Mn and Zn.
